# EUROPEAN PATENT SPECIFICATION

(11) **EP 1 442 711 B1**
(45) Date of publication and mention of the grant of the patent: **20.09.2006**
(21) Application number: 03257872.6
(22) Date of filing: 15.12.2003
(51) Int. Cl.: A61B 17/15

(54) **Bone jig**
Knochenlehre
Gabarit d'os

(30) Priority: 22.01.2003 GB 0301503
(43) Date of publication of application: 04.08.2004
(73) Proprietor: Biomet UK Limited, Bridgend South Wales CF31 3XA (GB)
(72) Inventor: Lennox, Iain Andrew Craig, Howe Green, Essex CM2 7TB (GB); Lloyd, Russel, Swindon, Wiltshire SN3 6EZ (GB); Metcalfe, Nick James Theophilus, Cardiff CF64 1DS (GB)
(74) Representative: Giles, Ashley Simon

(56) References cited:
- EP-A- 0 378 294
- WO-A-96/25114
- US-A- 6 013 081

## Description

This invention relates to a bone jig and particularly, although not exclusively, relates to a jig for preparing the distal end of a femur.

### BACKGROUND TO THE INVENTION

If a knee joint becomes damaged or diseased, it is known to replace the entire knee joint with a prosthesis. There are a large variety of different knee prostheses, but the most common type consists of a femoral component attached to the distal end of the femur and a separate tibial component attached to the proximal end of the tibia. These components can articulate directly on one another or can be separated by a meniscal bearing component. Where possible, all of the knee ligaments are retained, although in practice it is often necessary to remove at least the posterior cruciate ligament. It is desirable for the tension in the knee ligaments after surgery to be balanced throughout their range of motion.

The most complex component of a total knee prosthesis is the femoral component, since it carries not only the condylar bearing surfaces, but also the patella bearing surface which extends along an anterior face of the distal femur. Conventional femoral components require resecting of the distal end surface of the femur and the anterior and posterior faces of the femur. They also usually require two chamfered cuts to be made at the distal end of the femur anteriorly and posteriorly. The anterior/posterior position of the cuts made in the femur are vital in order to restore proper functioning of the knee and balance to the ligaments. Conventional jigs for resecting the femur use as a reference an intramedullary rod with a set anterior/posterior position on the jig on the anterior/posterior axis. The correct positioning of the jig is vitally important to ensuring equal tension in the ligaments after surgery, and the present invention has been devised with this in mind.

EP-A-0378294 discloses a jig for attachment to an end of a bone, the jig comprising a main body with at least one tool guide portion and a referencing member which attaches to the bone, the main body having adjusting means which acts between the main body and the referencing member to move the main body relative to the bone.

### STATEMENT OF INVENTION

According to the present invention, there is provided a jig as claimed in claim 1, in which the adjusting means comprises an adjusting screw, such as a pinion or worm, which engages a cooperating rack formed on the referencing member.

The adjusting means provides stepless translation of the main body relative to the referencing member and can be used to provide accurate adjustment of the tension in ligaments joining the first bone and a second bone.

Preferably, the adjusting screw is captive in the main body. Preferably, the adjusting screw is held captive in the main body by a retaining screw.

Preferably, the referencing member is pivotably connected to the main body, so that it can pivot in a plane, and the adjusting means moves the main body in a direction substantially perpendicular to the said plane. Preferably, when the jig is attached to a bone, the said plane is a varus/valgus plane and/or the said direction is an anterior/posterior direction.

Preferably, the tool guide portion of the jig comprises a slot for guiding the blade of a bone saw or other bone resecting device. The tool guide portion may also comprise a fitting to which a tool guide can be attached.

Preferably, the jig is adapted for resecting the human femur. The jig may further comprise an alignment device attached to the main body which is adapted to reference the jig with the anterior cortex of the femur.

Preferably, the adjusting means is provided with an overload device which prevents the adjusting means applying greater than a preset force to a joint.

Preferably, the overload device is set to slip at a preset tightening torque of the adjusting means.

Preferably, indicating means is provided to indicate the amount of adjustment and/or the force applied by the adjusting means.

Preferably, the main body is provided with drill guides, for forming peg holes in the bone.

Preferably, the referencing member comprises an intramedullary rod, which can be inserted into the medullary canal, thereby attaching the rod to the bone.

A jig in accordance with the present invention ensures accurate and repeatable positioning and alignment so that the tensions in the ligaments are balanced. This results in stability in the knee prosthesis throughout the range of active and passive motion. Such stability maintains constant contact pressure on the bearing to protect against subluxation and dislocation.

### BRIEF DESCRIPTION OF THE DRAWINGS

For a better understanding of the present invention, and to show more clearly how it may be carried into effect, reference will now be made, by way of example, to the accompanying drawings, in which:-
Figure 1 is a perspective view of a cutting jig (or contour block) having a screw adjustable intramedullary rod;
Figure 2 is a partially cut-away-view of the jig of Figure 1; and
Figure 3 shows the jig of Figure 1 attached to the distal end of a model of a human femur.

### DETAILED DESCRIPTION OF A PREFERRED EMBODIMENT

Referring to the Figures, a jig 2 for resecting a distal femur comprises a main body or "block" 4 having a plurality of tool guides 3, for guiding the blade of a bone saw (not shown). Passing through the block 4 are a first opening 6, and a second opening 8 which is parallel to the first opening 6. An anterior part 5 of the block 4 is provided with an anterior pocket 7 which is adapted to receive a calibrated stylus assembly 9 having a stylus point 11.

The first opening 6 comprises a cylindrical bore 10 which is integral with a slot 12 formed in a lower surface 14 of the block. Housed in the cylindrical bore 10 of the first opening 6 is a threaded pivot 16 which is connected to an intramedullary rod 18. The rod 18 passes through the slot 12 and projects downwardly from the lower surface 14 of the block.

The second opening 8 comprises a second cylindrical bore 20 disposed adjacent the first cylindrical bore 10. The first opening 6 and second opening 8 overlap to form a second slot 22 which extends between the first opening 6 and second opening 8. An adjusting screw 24 is located in the first opening 6, and threaded portions 26, 28 of the adjusting screw 24 engage the threaded pivot 16 through the second slot 22.

Between the threaded portions 26, 28, the adjusting screw 24 is waisted to form an annular groove 30. A grub screw 32, which is threaded into an upper part 34 of the block 4, projects into the annular groove 30 and holds the adjusting screw 24 captive.

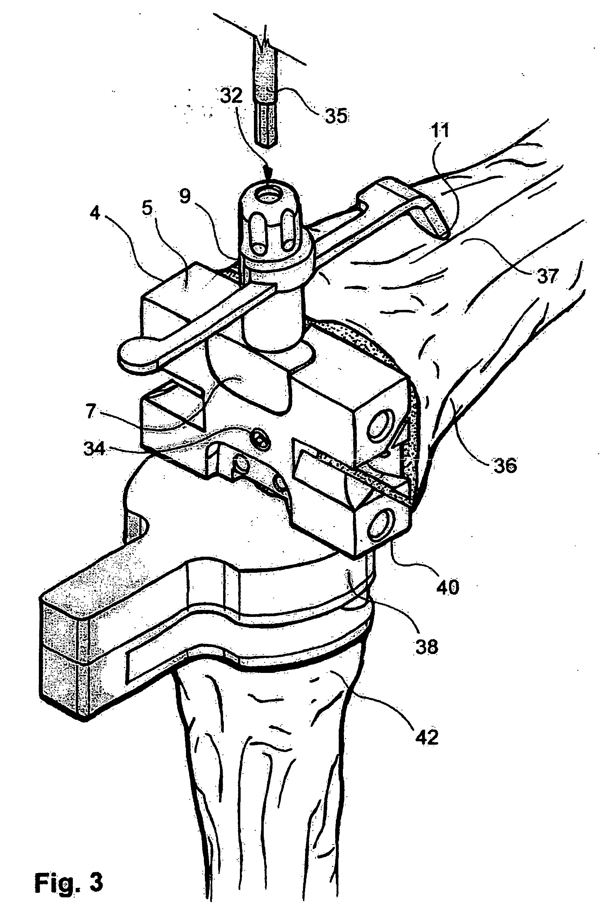

Referring particularly to Figure 3, the stylus assembly 9 is provided with a central bore which provides access for an Allen key 35 to be inserted into the adjusting screw 24.

In a surgical procedure to implant a total knee prosthesis, the proximal tibia 42 is resected using a standard technique, the distal femur 36 is cut off, and the medullary canal (not shown) which extends approximately along the longitudinal axis of the femur 36, is exposed. The intramedullary rod 18 of the jig 2 is inserted into the medullary canal such that the lower surface 14 of the block 4 contacts the resected upper surface of the distal femur 36. As the intramedullary rod 18 can pivot relative to the block 4, the lower surface 14 of the block can be brought into contact with the distal femur, whatever varus/valgus distal resection has been performed.

The stylus assembly 9 is inserted into the anterior pocket 7 of the block 4, and the block 4 is rotated about the longitudinal axis of the femur 36, until the stylus point 11 of the stylus assembly 9 is in contact with the anterior femoral cortex 37 of the femur 36. The stylus assembly 9 is kept aligned with the anterior femoral cortex 37 throughout the anterior/posterior adjustment of the block 4 to avoid the possibility of notching the distal femur when an anterior cut is made.

With the knee at 90 degrees of flexion, a spacer 38 (or tensor device - now shown) is inserted between a posterior surface 40 of the block 4 and the proximal end of the tibia 42. Preferably, the thickness of the spacer 38 is determined beforehand, with the leg in extension. An appropriate spacer 38 is selected such that it is closely received in the gap between the resected proximal tibia 42 and resected distal femur 36 in extension, when the required tension in the ligaments has been achieved and when the ligament tensions are balanced.

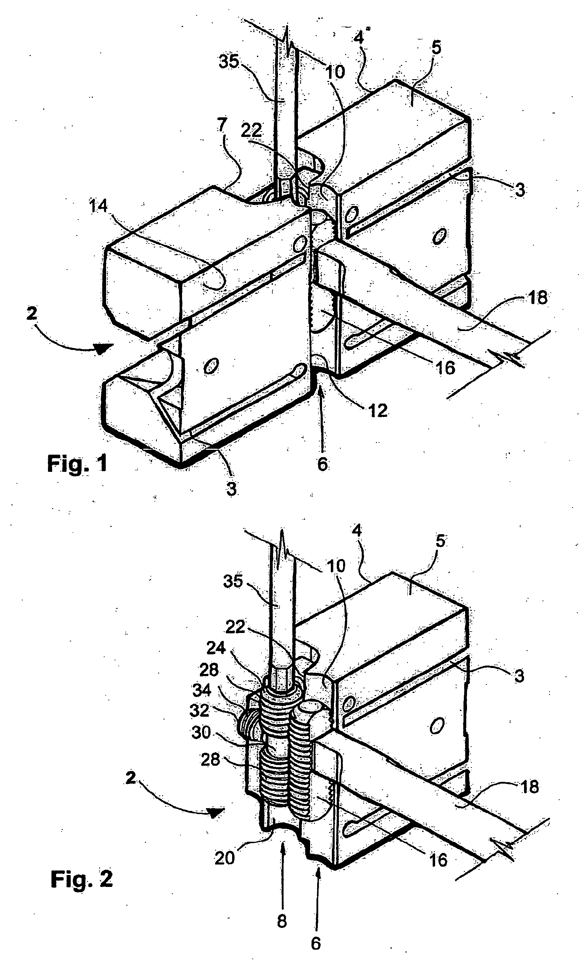

In order to accommodate the spacer 38 between the posterior surface 40 of the block 4 and the proximal tibia 42, the block 4 is moved in an anterior/posterior direction relative to the distal femur 36 by adjusting the adjusting screw 24. As best appreciated from Figure 2, the adjusting screw 24 is captive in the block 4 and the intramedullary rod 18 is fixed in the medullary canal of the femur 36. Consequently, as the adjusting screw 24 is rotated, it pulls itself along the threaded pivot 16, causing the block 4 to be moved in an anterior/posterior direction relative to the intramedullary rod 18 and distal femur 36.

The block 4 should be adjusted until it just seats flush against the spacer 38, thus providing the correct amount of external rotation of the block 4, according to the collateral ligament tension and the resected bone surfaces.

This allows the soft tissues to experience the same degree of tension as was present with the spacer 38 in extension, and assists in achieving correct rotational balance. If it is not possible to position the chosen block 4 adequately using translation of the block 4, the block 4 can be replaced with a block of a different size.

It should be noted that to avoid internal or incorrect rotation of the femur, it is important to address any soft tissue contractures prior to completing the positioning of the block 4. Marking Whitesides Line in the sulcus of the femur 36 can assist the visualisation of proper femoral rotation.

Once desired stability and good balance of the block 4 has been achieved, it is secured to the femur with bone nails or screws (not shown). Two cutting guides (not shown) are then clicked into position, so that anterior and posterior condyle cuts and chamfer cuts can be performed on the distal femur 36.

The adjusting screw 24 provides stepless adjustment, so that the desired gap between the posterior surface 40 of the block 4 and the proximal tibia 42 can be set accurately by reference to the spacer 38. If the block 4 is knocked or otherwise interfered with, it will not move in an anterior/posterior direction, since the adjusting screw 24 will not turn unless rotated positively by the Allen key 35.

Once adjusted in this way, the same quadrilateral gap, with similar ligament tensions, is achieved in flexion, to that in extension, and the collateral ligaments are in equilibrium (thereby ensuring that the correct amount of external rotation has been introduced).

## Claims

1. A jig (2) for use in preparing and/or resecting an end of a bone, the jig comprising a main body (4) with at least one tool guide portion (3) and a referencing member (18) which attaches to a bone (36), the main body (4) having adjusting means which acts between the main body (4) and the referencing member (18) to move the main body (4) relative to the bone (36), **characterised in that** the adjusting means comprises an adjusting screw (24)which engages a cooperating rack (16) formed on the referencing member.

2. A jig as claimed in claim 1, **characterised in that** the adjusting means is captive in the main body (4).

3. A jig as claimed in any one of the preceding claims, **characterised in that** the adjusting screw (24) is a pinion or worm.

4. A jig as claimed in any one of the preceding claims, **characterised in that** the referencing member (18) is pivotably connected to the main body (4), so that it can pivot in a plane and the adjusting means moves the main body (4) in a direction substantially perpendicular to the said plane.

5. A jig as claimed in claim 4, **characterised in that** when the main body is attached to a bone (36), the said plane is a varus/valgus plane and/or the said second direction is an anterior/posterior direction.

6. A jig as claimed in any one of the preceding claims, **characterised in that** the tool guide portion (3) comprises a slot for guiding the blade of a bone saw or other resecting device.

7. A jig as claimed in any one of claims 1 to 5, **characterised in that** the tool guide portion (3) is adapted to receive a removable tool guide.

8. A jig as claimed in any one of the preceding claims for use in resecting a human femur (36), the jig further **characterised by** an alignment device (9) attached to the main body (4) which is adapted to reference the jig with the anterior cortex of the distal femur.

9. A jig as claimed in any one of the preceding claims, **characterised in that** the adjusting means is provided with an overload device which prevents the adjusting means applying greater than a preset force to a joint.

10. A jig as claimed in claim 9, **characterised in that** the overload device is set to slip at a preset tightening torque of the adjusting means.

11. A jig as claimed in any one of the preceding claims, **characterised in that** indicating means is provided to indicate the amount of adjustment and/or the force applied by the adjusting means.

12. A jig as claimed in any one of the preceding claims, **characterised in that** the main body (4) is provided with drill guides, for forming peg holes in the bone (36).

13. A jig as claimed in any one of the preceding claims, **characterised in that** the referencing member (18) comprises an intramedullary rod.

## Patentansprüche

1. Lehre (2) zur Verwendung bei der Vorbereitung und/oder der Resektion eines Endes eines Knochens, wobei die Lehre einen Hauptkörper (4) mit mindestens einem Werkzeugführungsbereich (3) und einem Referenzteil (18) aufweist, der an einem Knochen (36) anbringbar ist, wobei der Hauptkörper (4) eine Einstelleinrichtung aufweist, die zwischen dem Hauptkörper (4) und dem Referenzteil (18) wirkt, um den Hauptkörper (4) in bezug auf den Knochen (36) zu bewegen, **dadurch gekennzeichnet, dass** die Einstelleinrichtung eine Einstellschraube (24) aufweist, welche an einer mit dieser zusammenwirkenden Zahnstange (16) angreift, die an dem Referenzteil (18) ausgebildet ist.

2. Lehre nach Anspruch 1, **dadurch gekennzeichnet, dass** die Einstelleinrichtung in dem Hauptkörper (4) unverlierbar gehalten ist.

3. Lehre nach einem der vorhergehenden Ansprüche, **dadurch gekennzeichnet, dass** die Einstellschraube (24) ein Ritzel oder eine Schnecke ist.

4. Lehre nach einem der vorhergehenden Ansprüche, **dadurch gekennzeichnet, dass** das Referenzteil (18) schwenkbar mit dem Hauptkörper (4) verbunden ist, so dass es in einer Ebene schwenken kann und die Einstelleinrichtung den Hauptkörper (4) im wesentlichen senkrecht zu dieser Ebene bewegt.

5. Lehre nach Anspruch 4, **dadurch gekennzeichnet, dass**, wenn der Hauptkörper an einem Knochen (36) angebracht ist, die Ebene eine Varus/Valgus-Ebene und/oder die zweite Richtung eine Anterior/Posterior-Richtung ist.

6. Lehre nach einem der vorhergehenden Ansprüche, **dadurch gekennzeichnet, dass** der Werkzeugführungsbereich (3) einen Schlitz zum Führen des Blatts einer Knochensäge oder einer anderen Resektionsvorrichtung aufweist.

7. Lehre nach einem der Ansprüche 1 bis 5, **dadurch gekennzeichnet, dass** der Werkzeugführungsbereich (3) zum Aufnehmen einer lösbaren Werkzeugführung ausgebildet ist.

8. Lehre nach einem der vorhergehenden Ansprüche zur Verwendung bei der Resektion eines menschlichen Oberschenkelknochens (36), wobei die Lehre ferner **gekennzeichnet ist durch** eine an dem Hauptkörper (4) angebrachte Ausrichtvorrichtung (9), die in der Lage ist, die Lehre in Ausrichtung auf die vordere Rinde des distalen Oberschenkelknochens zu bringen.

9. Lehre nach einem der vorhergehenden Ansprüche, **dadurch gekennzeichnet, dass** die Einstelleinrichtung mit einer Überlastungsvorrichtung versehen ist, welche verhindert, dass die Einstelleinrichtung einen höheren Druck als einen voreingestellten Druck auf ein Gelenk aufbringt.

10. Lehre nach Anspruch 9, **dadurch gekennzeichnet, dass** die Überlastungsvorrichtung derart eingestellt ist, dass sie bei einem voreingestellten Anzugsdrehmoment der Einstelleinrichtung durchrutscht.

11. Lehre nach einem der vorhergehenden Ansprüche, **dadurch gekennzeichnet, dass** eine Anzeigeeinrichtung vorgesehen ist, um den Einstellbetrag und/oder die von der Einstelleinrichtung aufgebrachte Kraft anzuzeigen.

12. Lehre nach einem der vorhergehenden Ansprüche, **dadurch gekennzeichnet, dass** der Hauptkörper (4) mit Bohrführungen zum Bilden von Stiftlöchern in dem Knochen (36) ausgebildet ist.

13. Lehre nach einem der vorhergehenden Ansprüche, **dadurch gekennzeichnet, dass** das Referenzteil (18) eine intramedullare Stange umfasst.

## Revendications

1. Gabarit (2) servant à apprêter et/ou réséquer une extrémité d'un os, le gabarit comprenant un corps principal (4) avec au moins une partie de guidage (3) d'outil et un élément de repérage (18) qui se fixe à un os (36), le corps principal (4) comportant un moyen de réglage qui agit entre le corps principal (4) et l'élément de repérage (18) pour déplacer le corps principal (4) par rapport à l'os (36), **caractérisé en ce que** le moyen de réglage comporte une vis de réglage (24) qui coopère avec une crémaillère (16) formée sur l'élément de repérage.

2. Gabarit selon la revendication 1, **caractérisé en ce que** le moyen de réglage est captif dans le corps principal (4).

3. Gabarit selon l'une quelconque des revendications précédentes, **caractérisé en ce que** la vis de réglage (24) est un pignon ou une vis sans fin.

4. Gabarit selon l'une quelconque des revendications précédentes, **caractérisé en ce que** l'élément de repérage (18) est monté de manière pivotante sur le corps principal (4) de façon à pouvoir pivoter dans un plan, et le moyen de réglage déplace le corps principal (4) dans une direction sensiblement perpendiculaire audit plan.

5. Gabarit selon la revendication 4, **caractérisé en ce que**, lorsque le corps principal est fixé à un os (36), ledit plan est un plan varus/valgus et/ou ladite seconde direction est une direction antéro-postérieure.

6. Gabarit selon l'une quelconque des revendications précédentes, **caractérisé en ce que** la partie de guidage (3) d'outil est constituée par une fente servant à guider la lame d'une scie à os ou autre dispositif de résection.

7. Gabarit selon l'une quelconque des revendications 1 à 5, **caractérisé en ce que** la partie de guidage (3) d'outil est conçue pour recevoir un guide d'outil amovible.

8. Gabarit selon l'une quelconque des revendications précédentes servant à la résection d'un fémur humain (36), le gabarit étant **caractérisé en outre par** un dispositif d'alignement (9) fixé au corps principal (4) qui sert à repérer le gabarit par rapport au cortex antérieur du fémur distal.

9. Gabarit selon l'une quelconque des revendications précédentes, **caractérisé en ce que** le moyen de réglage ést pourvu d'un dispositif de surcharge qui empêche le moyen de réglage d'appliquer à une articulation une force supérieure à une valeur préétablie.

10. Gabarit selon la revendication 9, **caractérisé en ce que** le dispositif de surcharge est conçu pour glisser à un couple de serrage préétabli du moyen de réglage.

11. Gabarit selon l'une quelconque des revendications précédentes, **caractérisé en ce qu'**un moyen d'indication est prévu pour indiquer l'ampleur du réglage et/ou de la force appliqué par le moyen de réglage.

12. Gabarit selon l'une quelconque des revendications précédentes, **caractérisé en ce que** le corps principal (4) est pourvu de guide-forets servant à former dans l'os (36) des trous pour des broches.

13. Gabarit selon l'une quelconque des revendications précédentes, **caractérisé en ce que** l'élément de repérage (18) comporte une tige intramédullaire.
